# EUROPÄISCHE PATENTSCHRIFT

(11) **EP 1 257 249 B1**
(45) Veröffentlichungstag und Bekanntmachung des Hinweises auf die Patenterteilung: **02.11.2006**
(21) Anmeldenummer: 01978366.1
(22) Anmeldetag: 13.09.2001
(51) Int. Cl.: A61K 8/49, A61Q 5/10

(54) **DIAMINOPYRAZOL-DERIVATE UND PYRAZOLON-DERIVATE ENTHALTENDE OXIDATIONSHAARFÄRBEMITTEL**
DIAMINOPYRAZOLE DERIVATIVES AND OXIDATION HAIR DYE CONTAINING PYRAZOLONE DERIVATIVES
SUBSTANCE DE COLORATION OXYDATIVE DES CHEVEUX CONTENANT DES DERIVES DE DIAMINOPYRAZOLE ET DES DERIVES DE PYRAZOLONE

(30) Priorität: 01.03.2001 DE 10109806
(43) Veröffentlichungstag der Anmeldung: 20.11.2002
(73) Patentinhaber: Wella Aktiengesellschaft, 64274 Darmstadt (DE)
(72) Erfinder: JAVET, Manuela, CH-1723 Marly (CH); DOUSSE, Christel, CH-1791 Courtaman (CH); LE CRUER, Dominique, CH-1723 Marly (CH)
(86) Internationale Anmeldenummer: PCT/EP2001/010585
(87) Internationale Veröffentlichungsnummer: WO 2002/069918

(56) Entgegenhaltungen:
- DE-A1- 19 916 033
- DE-U1- 20 013 156

## Beschreibung

Gegenstand der vorliegenden Erfindung sind Mittel zur oxidativen Färbung von Haaren, welche als Entwicklersubstanz bestimmte Diaminopyrazolderivate und als Kupplersubstanz bestimmte Pyrazolon-Derivate enthalten.

Haarfärbemittel werden je nach zu färbender Ausgangshaarfarbe und gewünschtem Endresultat hauptsächlich in den Bereich der Oxidationsfärbemittel und den Bereich der nicht-oxidativen Tönungen unterteilt. Oxidative Haarfarben haben heutzutage eine wesentliche Bedeutung erlangt. Die Färbung entsteht hierbei durch Reaktion von bestimmten Entwicklersubstanzen mit bestimmten Kupplersubstanzen in Gegenwart eines geeigneten Oxidationsmittels
Als Entwicklersubstanzen werden insbesondere 2,5-Diaminotoluol, 2,5-Diaminophenylethylalkohol, p-Aminophenol und 1,4-Diaminobenzol eingesetzt. Häufig eingesetzte Kupplerverbindungen sind: Resorcin, 1-Naphthol, 3-Aminophenol, 5-Amino-2-methylphenol, 4-Chlorresorcin und Derivate des m-Phenylendiamin.

Oxidationsfarbstoffe, die zur Färbung menschlicher Haare eingesetzt werden, müssen zahlreiche Anforderungen erfüllen. So müssen sie physiologisch verträglich sein und Färbungen in der jeweils gewünschten Intensität liefern. Ausserdem sollen die Haarfärbungen möglichst Beständig gegen die Einwirkung von Licht, Dauerwellmitteln und Säuren sowie Reibung sein und unter normalen Bedingungen mindestens 4 bis 6 Wochen stabil bleiben.

Ein oxidatives Färbesystem muss sowohl im Naturtonbereich als auch im Modetonbereich eine breite Palette verschiedener Farbnuancen ermöglichen. Das bedeutet, dass zum einen gelbe, rote und blaue Farbstoffe für den Modetonbereich und ausserdem blonde, braune und schwarze Farbstoffe für den Naturtonbereich zur Verfügung stehen. Die Naturtöne können aber auch gegebenenfalls aus verschiedenen Farbstoffen, die Gelb, Rot und Blau ergeben, gemischt werden.

Als Entwicklersubstanzen im Modetonbereich haben sich in neuerer Zeit physiologisch gut verträgliche 4,5-Diamino-pyrazol-Derivate durchgesetzt, die mit verschiedenen Kupplerverbindungen sehr intensive rote, violette und blau Farbtöne ergeben. Derartige Pyrazole enthaltende Oxidationsfärbemittel werden besipielsweise in der DE-OS 42 34 885, DE-OS 42 34 887, DE-PS 197 30 412, EP-OS 0 375 977 und EP-OS 0 740 931 beschrieben.

WO-A-02051372 (EP-A-1404288) beschreibt Haarfärbemittel, die 4,5-Diaminopyrazole als Entwickler, vorzugsweise 4,5-Diamino-1-hydroxyethylpyrazol, enthalten. 1-Phenyl-3-methylpyrazol-5-on wird als Kuppler vorgeschlagen.

DE-U-20013156 beschreibt Haarfärbemittel enthaltend 1,4-Diaminobenzol-Derivate und 4,5-Diaminopyrazol-Derivate, die im roten Farbbereich gute Ergebnisse geben.

Farbnuancen im Gelb und Orangebereich sind mit den vorstehend genannten Pyrazolderivaten jedoch bisher nicht beschrieben.

Aufgabe der vorliegenden Erfindung ist es daher, ein für den gelben und orangen Farbbereich geeignetes Entwicklersubstanz-Kupplersubstanz-System zur Verfügung zu stellen, welches als Entwicklersubstanz bestimmte Diaminopyrazolderivate enthält.

Es wurde nunmehr gefunden, dass diese Aufgabe durch den Einsatz von bestimmten Pyrazolon-Derivaten als Kupplersubstanz gelöst werden kann.

Gegenstand der vorliegenden Erfindung ist daher ein Mittel zur oxidativen Färbung von Keratinfasern -insbesondere Haaren-, welches dadurch gekennzeichnet ist, dass es (a) mindestens ein 4,5-Diaminopyrazol-Derivat der Formel (I), (II) oder (III) oder dessen Salz mit organischen oder anorganischen Säuren, 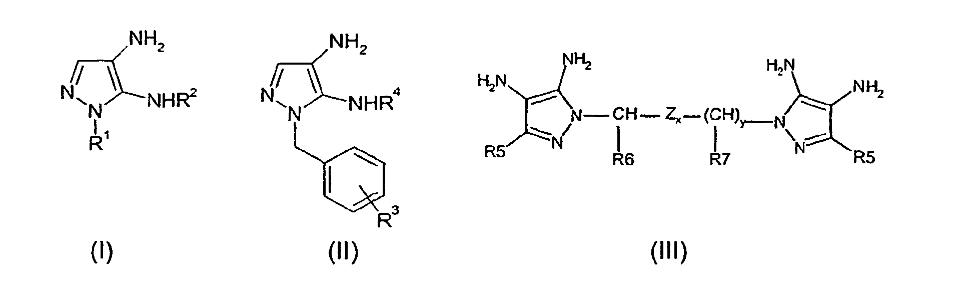
worin **R1** und **R2** unabhängig voneinander Wasserstoff, einen geradkettigen oder verzweigten C₁-C₆-Alkylrest, einen (gegebenenfalls substituierten) Phenylrest oder einen geradkettigen oder verzweigten C₂-C₄-Hydroxyalkylrest darstellen;
**R3** ein Halogenatom (F, Cl, Br, J), eine geradkettige oder verzweigte C₁-C₄-Alkylgruppe oder eine geradkettige oder verzweigte C₁-C₄-Alkoxygruppe ist und **R4** Wasserstoff, eine geradkettige oder verzweigte C₁-C₄₋Alkylgruppe oder eine geradkettige oder verzweigte C₂-C₄-Hydroxyalkylgruppe darstellt;
**R5** Wasserstoff, eine geradkettige oder verzweigte C₁-C₆-Alkylgruppe, eine C₁-C₄-Hydroxyalkylgruppe, eine C₁-C₄-Aminoalkylgruppe, eine C₁-C₈-Alkylaminogruppe, eine Di(C₁-C₈)-alkylaminogruppe, eine C₁-C₄₋Alkylamino-(C₁-C₄)alkylgruppe oder eine Di(C1-C4)-alkylamino-(C₁-C₄)-alkylgruppe, eine Arylgruppe oder eine Heteroarylgruppe darstellt;
**R6 und R7** gleich oder verschieden sein können und Wasserstoff, eine geradkettige oder verzweigte C₁-C₆-Alkylgruppe, eine Arylgruppe, eine Heteroarylgruppe, einer Carbonsäuregruppe, eine Carbonsäureester gruppe, eine unsubstituierte oder substituierte Carbonsäureamidgruppe, eine Hydroxygruppe oder eine C₁-C₄-Hydroxyalkylgruppe darstellen, oder aber R2 und R3 gemeinsam eine (gegebenenfalls substituierte) C₁-C₆₋Alkylengruppe bilden;
**Z** gleich einem, gegebenenfalls durch ein Heteroatom (beispielsweise ein Stickstoff-, Sauerstoff- oder Schwefel-Atom) unterbrochenen C₁-C₁₀-Alkyl-Diradikal, einem, gegebenenfalls ein- oder zweifach benzokondensierten und/oder mit einer Hydroxygruppe oder (C₁-C₆)-Alkylgruppe substituierten, aromatischen oder heteroaromatischen Diradikal, oder einem Diradikal der Formel **-Ar-(Alk)**_{**n**}**-Ar-** ist, wobei gilt **Ar** gleich einem(gegebenenfalls substituierten) Arylenrest oder Heteroarylenrest (insbesondere Phenylenrest oder Pyridylenrest), **Alk** gleich einer-CH₂-Gruppe und n gleich einer ganzen Zahl von 0 bis 6; und
**x** und **y** unabhängig voneinander jeweils gleich 0 oder 1 sind;
sowie (b) mindestens ein Pyrazol-5-on-Derivat der allgemeinen Formel (IV), (V) oder (VI), insbesondere (IV) oder (VI), oder dessen Salz mit organischen oder anorganischen Säuren, 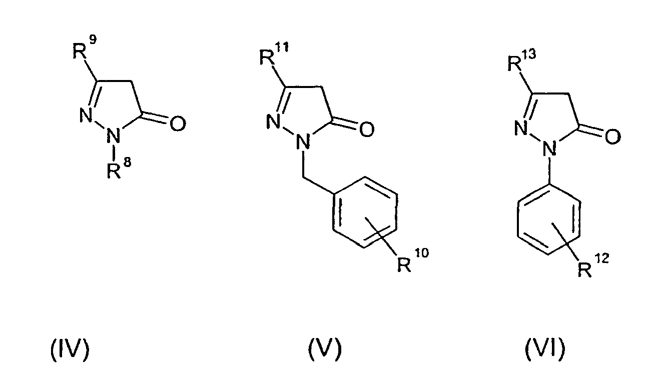 wobei
**R8** gleich einer geradkettigen oder verzweigten C₁-C₄-Alkylgruppe, einer geradkettigen oder verzweigten C₁-C₄-Alkoxygruppe, einer geradkettigen oder verzweigten C₁-C₄-Alkoxy-(C₁-C₄)alkylgruppe oder einer geradkettigen oder verzweigten C₂-C₄-Hydroxyalkylgruppe ist; und
die Reste **R9** bis **R13** unabhängig voneinander jeweils gleich Wasserstoff, einer geradkettigen oder verzweigten C₁-C₄-Alkylgruppe, einer geradkettigen oder verzweigten C₂-C₄-Hydroxyalkylgruppe, einer Hydroxygruppe, einer Aminogruppe, einer geradkettigen oder verzweigten C₁-C₄-Alkoxygruppe, einer geradkettigen oder verzweigten C₁-C₄-Alkoxy-(C₁-C₄)alkylgruppe oder einer C₂-C₄-Hydroxyalkylaminogruppe sind; enthält.

Als bevorzugte 4,5-Diaminopyrazol-Derivate der Formel (I), (II) und (III) sind insbesondere zu nennen: 4,5-Diamino-1-methyl-1H-pyrazol; 4,5-Diamino-1-(4'-methylbenzyl)-pyrazol; 4,5-Diamino-1-(3'-methylbenzyl)-pyrazol; 4,5-Diamino-1-(2'-methylbenzyl)-pyrazol; 4,5-Diamino-1-(2'-hydroxyethyl)-1 H-pyrazol; 4,5-Diamino-1-benzyl-1 H-pyrazol; 4,5-Diamino-1-ethyl-1 H-pyrazol; 4,5-Diamino-1-isopropyl-1H-pyrazol; 4,5-Diamino-1-pentyl-1 H-pyrazol; 4,5-Diamino-1-(4'-methoxy-benzyl)-1H-pyrazol; 4,5-Diamino-1 H-pyrazol; 4,5-Diamino-1-(3'-methoxy-benzyl)-1 H-pyrazol; 4,5-Diamino-1-(2'-methoxy-benzyl)-1H-pyrazol; 4,5-Diamino-1-(4'-chlorbenzyl)-1 H-pyrazol; 4,5-Diamino-1-(3'-chlorbenzyl)-1 H-pyrazol; 4,5-Diamino-1-(2'-chlorbenzyl)-1 H-pyrazol; 4-Amino-5-methylamino-1-(4'-methoxybenzyl)-1H-pyrazol; 4-Amino-5-(2'-hydroxyethyl)amino-1-(4'-methoxybenzyl)-1 H-pyrazol; 4-Amino-5-methylamino-1-(2'-hydroxyethyl)-1H-pyrazol; Bis-(4,5-diamino-pyrazol-1-yl)-methan; 1,2-Bis-(4,5-diamino-pyrazol-1-yl)-ethan; 1,3-Bis-(4,5-diamino-pyrazol-1-yl)-propan; 1,3-Bis-(4,5-diamino-3-phenyl-pyrazol-1-yl)-propan; 2,3-Bis-(4,5-diamino-pyrazol-1-yl)-propan-1-ol; N-Benzyl-2,3-bis-(4,5-diamino-pyrazol-1-yl)-propion-amid; 1,3-Bis-(4,5-diamino-pyrazol-1-yl)-cyclohexan; 1,4-Bis-(4,5-diamino-pyrazol-1-yl-methyl)-benzol; 1,4-Bis-(4,5-diamino-pyrazol-1-yl-methyl)-2,5-dimethoxy-benzol; 1,3-Bis-(4,5-diamino-pyrazol-1-yl-methyl)-benzol; 2,6-Bis-(4,5-diamino-pyrazol-1-yl-methyl)-4-methyl-phenol; 1,2-Bis-(4,5-diamino-pyrazol-1-yl-methyl)-benzol; 1,2-Bis -(4,5-diamino-pyrazol-1-yl-methyl)-4,5-dimethoxy-benzol; 2,3-Bis-(4,5-diamino-pyrazol-1-yl-methyl)-naphthalin; 2,3-Bis-(4,5-diamino-pyrazol-1-yl-methyl)-anthracen; 9,10-Bis-(4,5-diamino-pyrazol-1-yl-methyl)-anthracen, 4,4'-Bis-(4,5-diamino-pyrazol-1-yl-methyl)-biphenyl; 1,2-Bis-[4-(4,5-diamino-pyrazol-1-yl-methyl)-phenyl]-ethan; 2,5-Bis-(4,5-diamino-pyrazol-1-yl-methyl)-furan; 2,5-Bis-(4,5-diamino-pyrazol-1-yl-methyl)-thiophen; 2,8-Bis-(4,5-diamino-pyrazol-1-yl-methyl)-dibenzothiophen; 4,4'-Bis-(4,5-diamino-pyrazol-1-yl-methyl)-[2,2']bipyridyl und 1,2-Bis-[6-(4,5-diamino-pyrazol-1-yl-methyl)-pyridin-2-yl]-ethan oder deren Salze mit organischen oder anorganischen Säuren.

Als bevorzugte Pyrazolon-Derivate der Formeln (IV), (V) und (VI) sind insbesondere zu nennen: 3-Amino-1-phenyl-pyrazol-5-on, 3-Methyl-1-phenyl-pyrazol-5-on, 1,3-Dimethyl-pyrazol-5-on, 3-Methyl-1-(4-sulfphenyl)-pyrazol-5-on, 3-Methyl-1-(2-hydroxyethyl)- pyrazol-5-on und 1-Phenyl-pyrazol-5-on oder deren Salze mit organischen oder anorganischen Säuren.

Das 4,5-Diaminopyrazol der Formel (I), (II) oder (III) sowie das Pyrazolon-Derivat der Formeln (IV), (V) oder (VI) ist in dem erfindungsgemäßen Färbemittel jeweils in einer Gesamtmenge von etwa 0,005 bis 20 Gewichtsprozent enthalten, wobei eine Menge von etwa 0,01 bis 10 Gewichtsprozent und insbesondere 0,1 bis 6 Gewichtsprozent bevorzugt ist.

Das erfindungsgemäße Mittel kann neben den oben genannten 4,5-Diaminopyrazol-Derivaten auch noch weitere Entwicklersubstanzen enthalten. Besonders geeignet hierfür sind 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-2,5-dimethyl-benzol, 1,4-Diamino-2,3-dimethyl-benzol, 2-Chlor-1,4-diamino-benzol, 4-Phenylamino-anilin, 4-Dimethylamino-anilin, 4-Diethylamino-anilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1 H)-pyrimidon, 2-Amino-phenol, 2-Amino-6-methyl-phenol und 2-Amino-5-methyl-phenol oder deren Salze.

Außerdem kann die Farbträgermasse neben den oben genannten 2-Pyrazolon-5-on-Derivaten auch weitere zur Bildung einer Oxidationsfarbe geeignete Kupplersubstanzen enthalten. Hierfür können aromatische m-Diamine, m-Aminophenole, Polyphenole oder Naphthole eingesetzt werden. Besonders geeignet sind N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxy-ethoxy)-benzol, 2,4-Diamino-1-(3-hydroxypropoxy)-benzol, 2,4-Diamino-1-(3-methoxypropoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxy-ethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)-amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1 ,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 1,3-Dihydroxy-2,4-dimethyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 5,6-Dihydroxy-indol, 5,6-Dihydroxy-indolin, 4-Hydroxy-indol, 5-Hydroxy-indol, 6-Hydroxy-indol, 7-Hydroxy-indol und 2,3-Indolindion, oder deren Salze.

Die vorgenannten zusätzlichen Entwicklersubstanzen und Kuppler substanzen sind in dem Färbemittel jeweils in einer Gesamtmenge von etwa 0,01 bis 20 Gewichtsprozent, vorzugsweise 0,1 bis 10 Gewichtsprozent und insbesondere 0,1 bis 5 Gewichtsprozent, enthalten.

Weiterhin kann das Färbemittel gegebenenfalls zusätzlich übliche direktziehende anionische, kationische, zwitterionische oder nicht-ionische Farbstoffe enthalten. Zu den bevorzugten anionischen Farbstoffen zählen beispielsweise: 6-Hydroxy-5-[(4-sulfophenyl)azo]-2-naphthalinsulfonsäuredinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (CI47005;D&C Yellow No. 10; Food Yellow No. 13, Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)amino]-2-phenylamino-benzolsulfonsäurenatriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)-azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalindisulfonsäure-trinatriumsalz (Cl16185; Acid Red No. 27), 8-Amino-1-hydroxy-2-(phenylazo)-3,6-naphthalin-disulfonsäure-dinatriumsalz (Cl17200; Acid Red No. 33), 5-(Acetylamino)-4-hydroxy-3-[(2-methylphenyl)-azo]-2,7-naphthalin-disulfonsäure-dinatriumsalz (Cl18065; Acid Red No. 35), 2-(3-Hydroxy-2,4,5,7-tetraiod-dibenzopyran-6-on-9-yl)-benzoesäure-dinatriumsalz (Cl45430;Acid Red No. 51), N-[6-(Diethylamino)-9-(2,4-disulfophenyl)-3H-xanthen-3-yliden]-N-ethylethanaminium-hydroxid, inneres Salz, Natriumsalz (CI45100; Acid Red No. 52), 8-[(4-(Phenylazo)phenyl)azo]-7-naphthol-1,3-disulfonsäure-dinatriumsalz (Cl27290; Acid Red No. 73), 2',4',5',7'-Tetrabrom-3',6'-dihydroxyspiro[isobenzofuran-1 (3H),9'-[9H]xanthen]-3-on-dinatriumsalz (Cl45380; Acid Red No. 87), 2',4',5',7'-Tetrabrom-4,5,6,7-tetrachlor-3',6'-dihydroxyspiro[isobenzofuran-1 (3H),9'[9H]xanthen]-3-on-dinatriumsalz (Cl45410; Acid Red No. 92), 3',6'-Dihydroxy-4',5'-diiodospiro-[isobenzofuran-1 (3H),9'(9H)-xanthen)-3-on-dinatriumsalz (Cl45425; Acid Red No. 95), (2-Sulfophenyl)di[4-(ethyl((4-sulfophenyl)methyl)amino)-phenyl]-carbenium-dinatriumsalz, betain (Cl42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)-phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)-carbenium-inneres Salz, Natriumsalz (2:1) (CI42045; Food Blue No. 3; Acid Blue No. 1), Bis[4-(diethylamino)phenyl](5-hydroxy-2,4-disulfophenyl)-carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)-amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1) und 3-Hydroxy-4-(3-methyl-5-oxo-1-phenyl-4,5-dihydro-1 H-pyrazol-4-ylazo)-naphthalin-1-sulfonsäure Natriumsalz Chrom-Komplex (Acid Red No. 195). Zu den bevorzugten kationischen Farbstoffen zählen beispielsweise: 9-(Dimethylamino)-benzo[a]phenoxazin-7-ium-chlorid (CI51175; Basic Blue No. 6), Di[4-(diethylamino)phenyl][4-(ethylamino)-naphthyl]carbenium-chlorid (CI42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)-azo]-6-methoxy-3-methyl-benzothiazolium-methylsulfat (CI11154; Basic Blue No. 41), 8-Amino-2-brom-5-hydroxy-4-imino-6-[(3-(trimethyl-ammonio)phenyl)amino]-1(4H)-naphthalinon-chlorid (CI56059; Basic Blue No. 99), Bis[4-(dimethylamino)-phenyl][4-(methylamino)phenyl]carbenium-chlorid (Cl42535; Basic Violet No. 1), Tris(4-amino-3-methylphenyl)-carbenium-chlorid (Cl42520; Basic Violet No. 2), Tris[4-(dimethylamino)-phenyl]-carbenium-chlorid (Cl42555; Basic Violet No. 3), 2-[3,6-(Diethylamino)dibenzopyranium-9-yl]-benzoesäure-chlorid (Cl45170; Basic Violet No. 10), Di(4-aminophenyl)(4-amino-3-methylphenyl)carbenium-chlorid (Cl42510; Basic Violet No. 14), 1,3-Bis[(2,4-diamino-5-methylphenyl)azo]-3-methylbenzol (Cl21010;Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (CI12251; Basic Brown No. 17), 1-[(4-Amino-3-nitro-phenyl)azo]-7-(trimethylammonio)-2-naphthol-chlorid (Cl12251; Basic Brown No. 17), 3,7-Diamino-2,8-dimethyl-5-phenyl-phenazinium-chlorid (Cl50240; Basic Red No. 2), 1,4-Dimethyl-5-[(4-(dimethylamino)phenyl)azo]-1,2,4-triazolium-chlorid (Cl11055; Basic Red No. 22), 2-Hydroxy-1-[(2-methoxy-phenyl)azo]-7-(trimethylammonio)-naphthalin-chlorid (Cl12245; Basic Red No. 76), 2-[2-((2,4-Dimethoxyphenyl)amino)ethenyl]-1,3,3-trimethyl-3H-indol-1-ium-chlorid (Cl48055; Basic Yellow No. 11), 3-Methyl-1-phenyl-4-[(3-(trimethylammonio)phenyl)-azo]-pyrazol-5-on-chlorid (Cl12719; Basic Yellow No. 57) und Bis[4-(diethyl-amino)phenyl]phenylcarbenium-hydrogensulfat(1:1) (Cl42040; Basic Green No. 1). Als geeignete nichtionische Farbstoffe (insbesondere zum besseren Farbausgleich und zur Erzeugung von speziellen Nuancen können beispielsweise geannt werden: 1-Amino-2-[(2-hydroxyethyl)-amino]-5-nitrobenzol (HC Yellow No. 5), 1-(2-Hydroxyethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitro-phenol, 1-(2-Hydroxyethoxy)-3-methylamino-4-nitrobenzol, 2,3-(Dihydroxy-propoxy)-3-methylamino-4-nitrobenzol, 2-[(2-Hydroxyethyl)amino]-5-nitrophenol (HC Yellow No. 11), 3-[(2-Amino-ethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxy-ethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)-amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxy-ethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitro-benzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitro-phenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitro-pyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxy-ethyl)amino]-3-methylbenzol (HC Yellow No. 7), 2,6-Diamino-3-[(pyridin-3-yl)azo]-pyridin, 2-((4-(Acetylamino)phenyl)-azo)-4-methylphenol (CI11855; Disperse Yellow No. 3).

Aus der Gruppe der direktziehenden Farbstoffe besonders zu erwähnen sind 2-Amino-4,6-dinitrophenol, 2-Ethylamino-4,6-dinitrophenol, 2-[(2-Hydroxy-ethyl)amino]-4,6-dinitrophenol sowie Farbstoffe der allgemeinen Formel (VII), 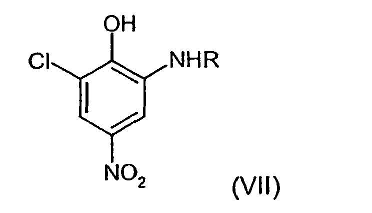 worin R Wasserstoff, Methyl, Ethyl oder Hydroxyethyl bedeutet.

Die direktziehenden Farbstoffe können in dem Färbemittel in einer Menge von etwa 0,01 bis 10 Gewichtsprozent, vorzugsweise 0,1 bis 5 Gewichtsprozent, eingesetzt werden.

Selbstverständlich können die Farbstoffe, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Die vorstehend beschriebenen erfindungsgemäßen Kombinationen der Verbindungen der Formeln (I) bis (III) und (IV) bis (VI) -sowie gegebenenfalls weiterer oxidativer Haarfarbstoffvorstufen und/oder direktziehender Farbstoffe- werden zur Färbung in einer geeigneten Farbträgermasse appliziert.

Darüber hinaus können in dem Färbemittel noch weitere übliche Zusatzstoffe, beispielsweise Antioxidantien wie Ascorbinsäure, Thioglykolsäure oder Natriumsulfit, sowie Parfümöle, Penetrationsmittel, Puffersysteme, Komplexbildner, Konservierungsstoffe, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,1 bis 30 Gewichtsprozent, die Verdicker in einer Menge von etwa 0,1 bis 30 Gewichtsprozent und die Pflegestoffe in einer Konzentration von etwa 0,1 bis 5,0 Gewichtsprozent.

Das gebrauchsfertige erfindungsgemäße Haarfärbemittel wird durch Mischen der Farbträgermasse mit einem Oxidationsmittel unmittelbar vor der Anwendung hergestellt.

Als Oxidationsmittel kommen hauptsächlich Wasserstoffperoxid oder dessen Additionsverbindungen an Harnstoff, Melamin, Natriumborat oder Natriumcarbonat in Form einer 1- bis 12prozentigen, vorzugsweise einer 3- bis 6prozentigen, wässrigen Lösung, in Betracht. Das Gewichtsverhältnis zwischen Haarfärbemittel und Oxidationsmittel beträgt hierbei vorzugsweise etwa 5:1 bis 1:3, insbesondere 1:1 bis 1:2. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Es ist prinzipiell auch möglich, zur Oxidation der Farbstoffe anstelle der vorgenannten Oxidationsmittel Luftsauerstoff zu verwenden.

Des pH-Wert des gebrauchsfertigen erfindungsgemäßen Haarfärbemittel stellt sich bei der Mischung der Farbträgermasse (deren pH-Wert etwa gleich 6 bis 11,5 ist) mit dem meist sauer eingestellten Oxidationsmittel (dessen pH-Wert etwa gleich 2 bis 6,5 ist) auf einen pH-Wert ein, der durch die Alkalimengen in der Farbträgermasse und die Säuremengen im Oxidationsmittel sowie durch das Mischungsverhältnis bestimmt wird.
Je nach Zusammensetzung kann das erfindungsgemäße Färbemittel schwach sauer, neutral oder alkalisch reagieren und im gebrauchsfertigen Zustand einen pH-Wert von etwa 3 und 11, vorzugsweise etwa 5 bis 10, aufweisen. Die basische Einstellung erfolgt hierbei vorzugsweise mit Ammoniak, wobei jedoch auch organische Amine, zum Beispiel 2-Amino-2-methyl-1-propanol, Tris(hydroxymethyl)amino-methan, Monoethanolamin und Triethanolamin, oder auch anorganische Basen wie Natriumhydroxid und Kaliumhydroxid Verwendung finden. Für eine pH-Wert-Einstellung im sauren Bereich kommen anorganische oder organische Säuren, zum Beispiel Phosphorsäure, Essigsäure, Milchsäure, Ascorbinsäure, Zitronensäure oder Weinsäure, in Betracht.

Anschliessend trägt man eine für die Haarfärbebehandlung ausreichende Menge, je nach Haarfülle, im allgemeinen etwa 60 bis 200 Gramm, dieses Gemisches auf das Haar auf und läßt das Gemisch bei etwa 15 bis 50 Grad Celsius, vorzugsweise 30 bis 40 Grad Celsius, etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das die erfindungsgemäße Kombination aus 4,5-Diaminopyrazolen der Formel (I), (II) oder (III) und Pyrazolon-Derivaten der Formel (IV), (V) oder (VI) enthaltende Färbemittel ermöglicht gelbe bis orangefarbene Haarfärbungen mit ausgezeichneter Farbechtheit, insbesondere was die Lichtechtheit, Waschechtheit und Reibeechtheit anbetrifft. Hierbei zeichnen sich die Farbtöne durch ihre besondere Farbintensität und Leuchtkraft aus.

Die nachfolgenden Beispiele sollen den Gegenstand näher erläutern, ohne ihn auf diese Beispiele zu beschränken.

### Beispiele

### Beispiel 1.1 bis 1.7: Haarfärbemittel

| | |
|---|---|
| 4,5-Diaminopyrazol-Derivat der | Mengenangaben |
| Formel (I) oder (II) | in Tabelle 1 |
| Pyrazol-5-on-Derivat der | Mengenangaben |
| Formel (IV) oder (VI) | in Tabelle 1 |
| Natriumhydroxyd (10 %ige wässrige Lösung) | 0,74 g |
| Natriumsulfit | 0,40 g |
| Ascorbinsäure | 0,30 g |
| Dinatrium-ethylendiaminotetraacetat | 0,30 g |
| Laurylethersulfat (28%ige wässrige Lösung) | 10,0 g |
| Ethanol | 8,00 g |
| Ammoniak (25 %ige wässrige Lösung) | 9,20 g |
| Wasser, vollentsalzt | ad 100,00 g |

5 g der vorstehenden Farbträgermasse werden mit 5 g einer 6 %igen Wasserstoffperoxidlösung vermischt. Das erhaltene gebrauchsfertige Oxidationshaarfärbemittel wird auf Haarsträhnchen aufgetragen und mit einem Pinsel gleichmäßig verteilt. Nach einer Einwirkungszeit von 20 Minuten bei 40 °C wird das Haar mit lauwarmem Wasser gespült und sodann getrocknet.

Die Färbeergebnisse sind in der nachfolgenden Tabelle 1 zusammengefaßt.

**Tabelle 1:**

| **Nr.** | **Entwickler-/Kupplerkombination** | **Farbton nach der Färbung** | **Farbmeßwerte der Haarsträhnchen** | | | |
|---|---|---|---|---|---|---|
| | | | | **L** | **a** | **b** |
| **1.1** | 4,5-Diamino-1-(2'-hydroxyethyl)-1 H-pyrazol sulfat: 2,30 g; 3-Methyl-1-phenyl-pyrazol-5-on: 1,74 g | | Vor dem Färben: | +83,30; | -0,48; | +10,40 |
| | | warmes gelb | Nach dem Färben: | +59,16; | +30,14; | +51,54 |
| **1.2** | 4,5-Diamino-1-(2'-hydroxyethyl)-1H-pyrazol sulfat: 2,30 g; 3-Amino-1-phenyl-pyrazol-5-on: 1,74 g | | Vor dem Färben: | +83,30; | -0,48; | +10,40 |
| | | gelb | Nach dem Färben: | +60,94; | +19,56; | +48,59 |
| **1.3** | 4,5-Diamino-1-(2'-hydroxyethyl)-1H-pyrazol sulfat: 2,30 g; 3-Methyl-1-(4-sulfophenyl)-pyrazol-5-on: 2,54 g | | Vor dem Färben: | +83,30; | -0,48; | +10,40 |
| | | gelb-orange | Nach dem Färben: | +51,54; | +34,11; | +37,34 |
| **1.4** | 4,5-Diamino-1-(2'-hydroxyethyl)-1 H-pyrazol sulfat: 2,30 g; 1,3-Dimethyl-pyrazol-5-on: 1,12 g | | Vor dem Färben: | +83,30; | -0,48; | +10,40 |
| | | gelb-orange | Nach dem Färben: | +58,29; | +30.96; | +45,53 |
| **1.5** | 4,5-Diamino-1-(2'-hydroxyethyl)-1H-pyrazol sulfat: 2,30 g; 3-Methyl-1-(2-hydroxyethyl)-pyrazol-5-on: 1,42 g | | Vordem Färben: | +83,30; | -0,48; | +10,40 |
| | | gelb-orange | Nach dem Färben: | +57,93; | +29,73; | +37,86 |
| **1.6** | 4,5-Diamino-1-(4'-methylbenzyl)-1H-pyrazol sulfat: 2,41 g; 3-Methyl-1-phenyl-pyrazol-5-on: 1,74 g | | Vor dem Färben: | +83,30; | -0,48; | +10,40 |
| | | orange | Nach dem Färben: | +56,25; | +33,04; | +51,02 |
| **1.7** | Entwickler: | | Vor dem Färben: | +83,30; | -0,48; | +10,40 |
| | 4,5-Diamino-1-(2'-hydroxyethyl)-1H-pyrazol sulfat: 2,30 g; | braun | Nach dem Färben: | +27,91; | +22,30; | +8,29 |
| | Kuppler: | | | | | |
| | 3-Amino-1-phenyl-pyrazol-5-on: 1,31 g N-(3-(Dimethylamino)phenyl)-harnstoff: 0,42 4-Amino-2-hydroxy-toluol: 0,008 g | | | | | |

### Beispiel 2.1 bis 2.6: Haarfärbemittel

| | |
|---|---|
| Verbrücktes Diaminopyrazol-Derivat der | Mengenangaben |
| Formel (III) | in Tabelle 1 |
| Pyrazol-5-on-Derivat der Formel (IV) oder (VI) | Mengenangaben |
| | in Tabelle 1 |
| Natriumhydroxyd (10 %ige wässrige Lösung) | 0,74 g |
| Natriumsulfit | 0,40 g |
| Ascorbinsäure | 0,30 g |
| Dinatrium-ethylendiaminotetraacetat | 0,30 g |
| Laurylethersulfat (28%ige wässrige Lösung) | 10,0 g |
| Ethanol | 8,00 g |
| Ammoniak (25 %ige wässrige Lösung) | 9,20 g |
| Wasser, vollentsalzt | ad 100,0 g |

5 g der vorstehenden Farbträgermasse werden mit 5 g einer 6 %igen Wasserstoffperoxidlösung vermischt. Das erhaltene gebrauchsfertige Oxidationshaarfärbemittel wird auf Haarsträhnchen aufgetragen und mit einem Pinsel gleichmäßig verteilt. Nach einer Einwirkungszeit von 20 Minuten bei 40 °C wird das Haar mit lauwarmem Wasser gespült und sodann getrocknet.

Die Färbeergebnisse sind in der nachfolgenden Tabelle 1 zusammengefaßt.

**Tabelle 1:**

| **Nr.** | **Entwickler-/Kupplerkombination** | **Farbton nach der Färbung** | **Farbmeßwerte der Haarsträhnchen** | | | |
|---|---|---|---|---|---|---|
| | | | | **L** | **a** | **b** |
| **2.1** | 1,4-Bis-(4,5-diamino-pyrazol-1-yl-methyl)-benzol: 4,28 g; 3-Methyl-1-phenyl-pyrazol-5-on: 1,74 g | | Vor dem Färben: | +83,30; | -0,48; | +10,40 |
| | | warmes gelb | Nach dem Färben: | +57,05; | +31,30; | +50,43 |
| | | | Waschtest: Farbergebnis nach 5h in demineralisiertem Wasser: | +50,14; | +31,87; | +45,33 |
| **2.2** | 1,4-Bis-(4,5-diamino-pyrazol-1-yl-methyl)-benzol: 4,28 g; 3-Amino-1-phenyl-pyrazol-5-on: 1,75 g | | Vor dem Färben: | +83,30; | -0,48; | +10,40 |
| | | gelb | Nach dem Färben: | +63,78; | +15,33; | +53,29 |
| | | | Waschtest: Farbergebnis nach 5h in demineralisiertem Wasser: | +51,56; | +23,09; | +44,59 |
| **2.3** | 1,2-Bis-(4,5-diamino-pyrazol-1-yl)- ethan: 3,52 g; 3-Methyl-1-phenyl-pyrazol-5-on: 1,74 g | | Vor dem Färben: | +83,30; | -0,48; | +10,40 |
| | | braungelb | Nach dem Färben: | +54,04; | +12,46; | +36,56 |
| | | | Waschtest: Farbergebnis nach 5h in demineralisiertem Wasser: | 45,86; | +15,44; | +32,04 |
| **2.4** | 1,2-Bis-(4,5-diamino-pyrazol-1-yl)- ethan: 3,52 g; 3-Amino-1-phenyl-pyrazol-5-on: 1,75 g | | Vor dem Färben: | +83,30; | -0,48; | +10,40 |
| | | grüngelb | Nach dem Färben: | +62,35; | +10,76; | +37,69 |
| | | | Waschtest: Farbergebnis nach 5h in deminerali- siertem Wasser: | 46,14; | +8,65; | +31,24 |
| **2.5** | 1,4-Bis-(4,5-diamino-pyrazol-1-yl-methyl)-benzol: 4,28 g; 1,3-Dimethyl-pyrazol-5-on: 1,12 g | | Vor dem Färben: | +83,30; | -0,48; | +10,40 |
| | | warmes gelb | Nach dem Färben: | +50,76; | +32,70; | +45,70 |
| | | | Waschtest: Farbergebnis nach 5h in demineralisiertem Wasser: | 43,77; | +32,41; | +35,82 |
| **2.6** | 1,2-Bis-(4,5-diamino-pyrazol-1-yl)-ethan: 3,52 g; 1,3-Dimethyl-pyrazol-5-on: 1,12 g | | Vor dem Färben: | +83,30; | -0,48; | +10,40 |
| | | mittelblond | Nach dem Färben: | +47,91; | +32,41; | +35;82 |
| | | | Waschtest: Farbergebnis nach 5h in demineralisiertem Wasser: | 40,04; | +12,68; | +20,85 |

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

## Patentansprüche

1. Mittel zur oxidativen Färbung von Keratinfasem, **dadurch gekennzeichnet, dass** es (a) mindestens ein 4,5-Diaminopyrazol-Derivat der Formel (I), (II) oder (III) oder dessen Salz mit organischen oder anorganischen Säuren, 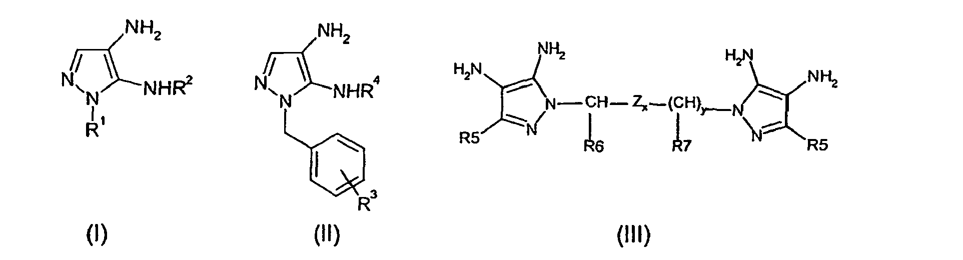
worin **R1** und **R2** unabhängig voneinander Wasserstoff, einen geradkettigen oder verzweigten C₁-C₆-Alkylrest, einen (gegebenenfalls substituierten) Phenylrest oder einen geradkettigen oder verzweigten C₂-C₄-Hydroxyalkylrest darstellen;
**R3** ein Halogenatom, eine geradkettige oder verzweigte C₁-C₄-Alkylgruppe oder eine geradkettige oder verzweigte C₁-C₄-Alkoxygruppe ist und R4 Wasserstoff, eine geradkettige oder verzweigte C₁-C₄-Alkylgruppe oder eine geradkettige oder verzweigte C₂-C₄-Hydroxyalkylgruppe darstellt;
**R5** Wasserstoff, eine geradkettige oder verzweigte C₁-C₈-Alkylgruppe, eine C₁-C₄-Hydroxyalkylgruppe, eine C₁-C₄-Aminoalkylgruppe, eine C₁-C₆-Alkylaminogruppe, eine Di(C₁-C₈)-alkylaminogruppe, eine C₁-C₄₋Alkylamino-(C₁-C₄)alkylgruppe oder eine Di(C1-C4)-alkylamino-(C₁-C₄)-alkylgruppe, eine Arylgruppe oder eine Heteroarylgruppe darstellt;
**R6 und R7** gleich oder verschieden sein können und Wasserstoff, eine geradkettige oder verzweigte C₁-C₆-Alkylgruppe, eine Arylgruppe, eine Heteroarylgruppe, einer Carbonsäuregruppe, eine Carbonsäureester gruppe, eine unsubstituierte oder substituierte Carbonsäureamidgruppe, eine Hydroxygruppe oder eine C₁-C₄-Hydroxyalkylgruppe darstellen, oder aber R2 und R3 gemeinsam eine (gegebenenfalls substituierte) C₁-C₆₋Alkylengruppe bilden;
**Z** gleich einem, gegebenenfalls durch ein Heteroatom unterbrochenen C₁-C₁₀-Alkyl-Diradikal, einem, gegebenenfalls ein- oder zweifach benzokondensierten und/oder mit einer Hydroxygruppe oder (C₁-C₆)-Alkylgruppe substituierten, aromatischen oder heteroaromatischen Diradikal, oder einem Diradikal der Formel -Ar-(Alk)ₙ-Ar- ist, wobei gilt Ar gleich einem (gegebenenfalls substituierten) Arylenrest oder Heteroarylenrest, Alk gleich einer ―CH₂-Gruppe und n gleich einer ganzen Zahl von 0 bis 6; und
**x** und **y** unabhängig voneinander jeweils gleich 0 oder 1 sind;
sowie (b) mindestens ein Pyrazol-5-on-Derivat der allgemeinen Formel (IV), (V) oder (VI), insbesondere (IV) oder (VI), oder dessen Salz mit organischen oder anorganischen Säuren, 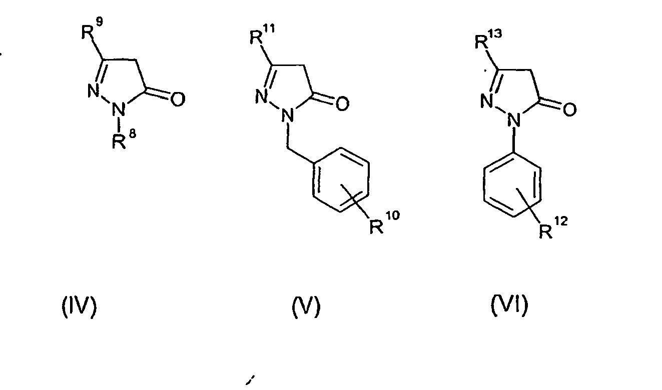 wobei
**R8** gleich einer geradkettigen oder verzweigten C₁-C₄-Alkylgruppe, einer geradkettigen oder verzweigten C₁-C₄-Alkoxygruppe, einer geradkettigen oder verzweigten C₁-C₄-Alkoxy-(C₁-C₄)alkylgruppe oder einer geradkettigen oder verzweigten C₂-C₄-Hydroxyalkylgruppe ist; und
die Reste **R9** bis **R13** unabhängig voneinander jeweils gleich Wasserstoff, einer geradkettigen oder verzweigten C₁-C₄-Alkylgruppe, einer geradkettigen oder verzweigten C₂-C₄-Hydroxyalkylgruppe, einer Hydroxygruppe, einer Aminogruppe, einer geradkettigen oder verzweigten C₁-C₄-Alkoxygruppe, einer geradkettigen oder verzweigten C₁-C₄-Alkoxy-(C₁-C₄)alkylgruppe oder einer C₂-C₄-Hydroxyalkylaminogruppe sind; enthält.

2. Mittel nach Anspruch 1, **dadurch gekennzeichnet, dass** das 4,5-Diaminopyrazol-Derivat der Formel (I), (II) oder (III) ausgewählt ist aus 4,5-Diamino-1-methyl-1 H-pyrazol; 4,5-Diamino-1-(4'-methylbenzyl)-pyrazol; 4,5-Diamino-1-(3'-methylbenzyl)-pyrazol; 4,5-Diamino-1-(2'-methylbenzyl)-pyrazol; 4,5-Diamino-1-(2'-hydroxyethyl)-1H-pyrazol; 4,5-Diamino-1-benzyl-1 H-pyrazol; 4,5-Diamino-1-ethyl-1 H-pyrazol; 4,5-Diamino-1-isopropyl-1H-pyrazol; 4,5-Diamino-1-pentyl-1H-pyrazol; 4,5-Diamino-1-(4'-methoxy-benzyl)-1H-pyrazol; 4,5-Diamino-1H-pyrazol; 4,5-Diamino-1-(3'-methoxy-benzyl)-1H-pyrazol; 4,5-Diamino-1-(2'-methoxy-benzyl)-1 H-pyrazol; 4,5-Diamino-1-(4'-chlor-benzyl)-1H-pyrazol; 4,5-Diamino-1-(3'-chlorbenzyl)-1H-pyrazol; 4,5-Diamino-1-(2'-chlorbenzyl)-1H-pyrazol; 4-Amino-5-methylamino-1-(4'-methoxybenzyl)-1H-pyrazol; 4-Amino-5-(2'-hydroxyethyl)amino-1-(4'-methoxybenzyl)-1H-pyrazol; 4-Amino-5-methylamino-1-(2'-hydroxyethyl)-1H-pyrazol; Bis-(4,5-diamino-pyrazol-1-yl)-methan; 1,2-Bis-(4,5-diamino-pyrazol-1-yl)-ethan; 1,3-Bis-(4,5-diamino-pyrazol-1-yl)-propan; 1,3-Bis-(4,5-diamino-3-phenyl-pyrazol-1-yl)-propan; 2,3-Bis-(4,5-diamino-pyrazol-1-yl)-propan-1-ol; N-Benzyl-2,3-bis-(4,5-diamino-pyrazol-1-yl)-propionamid; 1,3-Bis-(4,5-diamino-pyrazol-1-yl)-cyclohexan; 1,4-Bis-(4,5-diamino-pyrazol-1-yl-methyl)-benzol; 1,4-Bis-(4,5-diamino-pyrazol-1-yl-methyl)-2,5-dimethoxy-benzol; 1,3-Bis-(4,5-diamino-pyrazol-1-yl-methyl)-benzol; 2,6-Bis-(4,5-diamino-pyrazol-1-yl-methyl)-4-methyl-phenol; 1,2-Bis -(4,5-diamino-pyrazol-1-yl-methyl)-benzol; 1,2-Bis -(4,5-diamino-pyrazol-1-yl-methyl)-4,5-dimethoxy-benzol; 2,3-Bis-(4,5-diamino-pyrazol-1-yl-methyl)-naphthalin; 2,3-Bis-(4,5-diamino-pyrazol-1-yl-methyl)-anthracen; 9,10-Bis-(4,5-diamino-pyrazol-1-yl-methyl)-anthracen, 4,4'-Bis-(4,5-diamino-pyrazol-1-yl-methyl)-biphenyl; 1,2-Bis-[4-(4,5-diamino-pyrazol-1-yl-methyl)-phenyl]-ethan; 2,5-Bis-(4,5-diamino-pyrazol-1-yl-methyl)-furan; 2,5-Bis-(4,5-diamino-pyrazol-1-yl-methyl)-thiophen; 2,8-Bis-(4,5-diamino-pyrazol-1-yl-methyl)-dibenzothiophen; 4,4'-Bis-(4,5-diamino-pyrazol-1-yl-methyl)-[2,2']bipyridyl und 1,2-Bis-[6-(4,5-diamino-pyrazol-1-yl-methyl)-pyridin-2-yl)-ethan oder deren Salzen mit organischen oder anorganischen Säuren.

3. Mittel nach Anspruch 1, **dadurch gekennzeichnet, dass** das Pyrazolon-Derivat der Formel (IV), (V) oder (VI) ausgewählt ist aus 3-Amino-1-phenyl-pyrazol-5-on, 3-Methyl-1-phenyl-pyrazol-5-on, 1,3-Dimethyl-pyrazol-5-on, 3-Methyl-1-(4-sulfphenyl)- pyrazol-5-on, 3-Methyl-1-(2-hydroxyethyl)- pyrazol-5-on und 1-Phenyl-pyrazol-5-on oder deren Salzen mit organischen oder anorganischen Säuren.

4. Mittel nach einem der Ansprüche 1 bis 3, **dadurch gekennzeichnet, dass** es das 4,5-Diaminopyrazol der Formel (I), (II) oder (III) und das Pyrazolon-Derivat der Formel (IV), (V) oder (VI) jeweils in einer Gesamtmenge von 0,005 bis 20 Gewichtsprozent enthält.

5. Mittel nach einem der Ansprüche 1 bis 4, **dadurch gekennzeichnet, dass** es zusätzlich zu den Farbstoffen der Formeln (I) bis (VI) weitere oxidative Farbstoffvorstufen (Entwicklersubstanzen und/oder Kupplersubstanzen) und/oder direktziehende Farbstoffe enthält.

6. Mittel nach Anspruch 5, **dadurch gekennzeichnet, dass** es die zusätzlichen Entwicklersubstanzen und Kupplersubstanzen jeweils in einer Menge von 0,01 bis 20 Gewichtsprozent enthält.

7. Mittel nach Anspruch 5, **dadurch gekennzeichnet, dass** es die direktziehenden Farbstoffe in einer Menge von 0,1 bis 10 Gewichtsprozent enthält.

8. Mittel nach einem der Ansprüche 1 bis 7, **dadurch gekennzeichnet, dass** es vor der Anwendung mit dem Oxidationsmittel in einem Gewichtsverhältnis von 5:1 bis 1:3 vermischt wird.

9. Mittel nach Anspruch 8, **dadurch gekennzeichnet, dass** das gebrauchsfertige Oxidationsfärbemittel einen pH-Wert von 3 bis 11 aufweist.

## Claims

1. Agent for the oxidative colouring of keratin fibres, **characterized in that** it comprises (a) at least one 4,5-diaminopyrazole derivative of the formula (I), (II) or (III) or salts thereof with organic or inorganic acids, 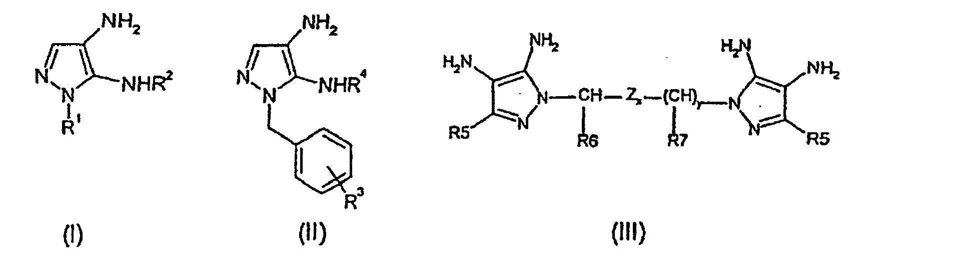
in which **R1** and **R2,** independently of one another, are hydrogen, a straight-chain or branched C₁-C₈-alkyl radical, an (optionally substituted) phenyl radical or a straight-chain or branched C₂-C₄-hydroxyalkyl radical;
**R3** is a halogen atom, a straight-chain or branched C₁-C₄-alkyl group or a straight-chain or branched C₁-C₄₋alkoxy group, and **R4** is hydrogen, a straight-chain or branched C₁-C₄-alkyl group or a straight-chain or branched C₂-C₄-hydroxyalkyl group;
**R5** is hydrogen, a straight-chain or branched C₁-C₆-alkyl group, a C₁-C₄-hydroxyalkyl group, a C₁-C₄-aminoalkyl group, a C₁-C₈-alkylamino group, a di (C₁-C₈) alkylamino group, a C₁-C₄-alkylamino (C₁-C₄) alkyl group or a di(C₁-C₄)alkylamino(C₁-C₄)alkyl group, an aryl group or a heteroaryl group;
**R6** and **R7** may be identical or different and are hydrogen, a straight-chain or branched C₁-C₆-alkyl group, an aryl group, a heteroaryl group, a carboxylic acid group, a carboxylic ester group, an unsubstituted or substituted carboxamide group, a hydroxy group or a C₁-C₄-hydroxyalkyl group, or else R2 and R3 together form an (optionally substituted) C₁-C₆-alkylene group;
Z is a C₁-C₁₀-alkyl diradical optionally interrupted by a heteroatom, an aromatic or heteroaromatic diradical optionally mono- or dibenzofused and/or substituted by a hydroxy group or (C₁-C₆)alkyl group, or a diradical of the formula **-Ar-(Alk)**_{**n**}**-Ar-** where **Ar** is an (optionally substituted) arylene radical or heteroarylene radical, **Alk** is a -CH₂ group and **n** is an integer from 0 to 6; and
**x** and **y**, independently of one another, are each 0 or 1;
and (b) at least one pyrazol-5-one derivative of the general formula (IV), (V) or (VI), in particular (IV) or (VI); or salt thereof with organic or inorganic acids, 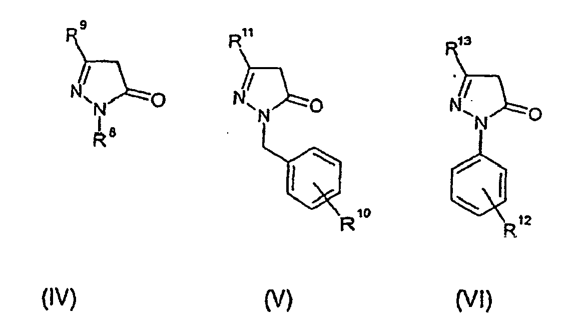 where
**R8** is a straight-chain or branched C₁-C₄-alkyl group, a straight-chain or branched C₁-C₄-alkoxy group, a straight-chain or branched C₁-C₄-alkoxy (C₁-C₄) alkyl group or a straight-chain or branched C₂-C₄-hydroxyalkyl group; and
the radicals **R9** to **R13**, independently of one another, are each hydrogen, a straight-chain or branched C₁-C₄₋alkyl group, a straight-chain or branched C₂-C₄-hydroxyalkyl group, a hydroxy group, an amino group, a straight-chain or branched C₁-C₄-alkoxy group, a straight-chain or branched C₁-C₄-alkoxy(C₁-C₄)alkyl group or a C₂-C₄-hydroxyalkylamino group.

2. Agent according to Claim 1, **characterized in that** the 4,5-diaminopyrazole derivative of the formula (I), (II) or (III) is chosen from 4,5-diamino-1-methyl-1H-pyrazole; 4,5-diamino-1-(4'-methylbenzyl)pyrazole; 4,5-diamino-1-(3'-methylbenzyl)pyrazole; 4,5-diamino-1-(2'-methylbenzyl)pyrazole; 4,5-diamino-1-(2'-hydroxyethyl)-1H-pyrazole; 4,5-diamino-1-benzyl-1H-pyrazole; 4,5-diamino-1-ethyl-1H-pyrazole; 4,5-diamino-1-isopropyl-1H-pyrazole; 4,5-diamino-1-pentyl-1H-pyrazole; 4,5-diamino-1-(4'-methoxybenzyl)-1H-pyrazole; 4,5-diamino-1H-pyrazole; 4,5-diamino-1-(3'-methoxybenzyl)-1H-pyrazole; 4,5-diamino-1-(2'-methoxybenzyl)-1H-pyrazole; 4,5-diamino-1-(4'-chlorobenzyl)-1H-pyrazole; 4,5-diamino-1-(3'-chlorobenzyl)-1H-pyrazole; 4,5-diamino-1-(2'-chlorobenzyl)-1H-pyrazole; 4-amino-5-methylamino-1-(4'-methoxybenzyl)-1H-pyrazole; 4-amino-5-(2'-hydroxyethyl)amino-1-(4'-methoxybenzyl)-1H-pyrazole; 4-amino-5-methylamino-1-(2'-hydroxyethyl)-1H-pyrazole; bis(4,5-diaminopyrazol-1-yl)methane; 1,2-bis-(4,5-diaminopyrazol-1-yl)ethane; 1,3-bis(4,5-diamino-pyrazol-1-yl)propane; 1,3-bis(4,5-diamino-3-phenyl-pyrazol-1-yl)propane; 2,3-bis(4,5-diaminopyrazol-1-yl)-propan-1-ol; N-benzyl-2,3-bis(4,5-diaminopyrazol-1-yl)-propionamide; 1,3-bis(4,5-diaminopyrazol-1-yl)cyclohexane; 1,4-bis(4,5-diaminopyrazol-1-ylmethyl)benzene; 1,4-bis(4,5-diaminopyrazol-1-ylmethyl)-2,5-dimethoxy-benzene; 1,3-bis(4,5-diaminopyrazol-1-ylmethyl)benzene; 2,6-bis(4,5-diaminopyrazol-1-ylmethyl)-4-methylphenol; 1,2-bis(4,5-diaminopyrazol-1-ylmethyl)benzene; 1,2-bis-(4,5-diaminopyrazol-1-ylmethyl)-4,5-dimethoxybenzene; 2,3-bis(4,5-diaminopyrazol-1-ylmethyl)naphthalene; 2,3-bis(4,5-diaminopyrazol-1-ylmethyl)anthracene; 9,10-bis(4,5-diaminopyrazol-1-ylmethyl)anthracene, 4,4'-bis(4,5-diaminopyrazol-1-ylmethyl)biphenyl; 1,2-bis[4-(4,5-diaminopyrazol-1-ylmethyl)phenyl]ethane; 2,5-bis(4,5-diaminopyrazol-1-ylmethyl)furan; 2,5-bis-(4,5-diaminopyrazol-1-ylmethyl)thiophene; 2,8-bis(4,5-diaminopyrazol-1-ylmethyl)dibenzothiophene; 4,4'-bis-(4,5-diaminopyrazol-1-ylmethyl)-[2,2']bipyridyl and 1,2-bis[6-(4,5-diaminopyrazol-1-ylmethyl)pyridin-2-yl]-ethane or salts thereof with organic or inorganic acids.

3. Agent according to Claim 1, **characterized in that** the pyrazolone derivative of the formula (IV), (V) or (VI) is chosen from 3-amino-1-phenylpyrazol-5-one, 3-methyl-1-phenylpyrazol-5-one, 1,3-dimethylpyrazol-5-one, 3-methyl-1-(4-sulphophenyl)pyrazol-5-one, 3-methyl-1-(2-hydroxyethyl)pyrazol-5-one and 1-phenyl-pyrazol-5-one or salts thereof with organic or inorganic acids.

4. Agent according to one of Claims 1 to 3, **characterized in that** it comprises the 4,5-diaminopyrazole of the formula (I), (II) or (III) and the pyrazolone derivative of the formula (IV), (V) or (VI) in each case in a total amount of from 0.005 to 20 per cent by weight.

5. Agent according to one of Claims 1 to 4, **characterized in that**, in addition to the dyes of the formulae (I) to (VI), it comprises further oxidative dye precursors (developer substances and/or coupler substances) and/or direct dyes.

6. Agent according to Claim 5, **characterized in that** it comprises the additional developer substances and coupler substances in each case in an amount of from 0.01 to 20 per cent by weight.

7. Agent according to Claim 5, **characterized in that** it comprises the direct dyes in an amount of from 0.1 to 10 per cent by weight.

8. Agent according to one of Claims 1 to 7, **characterized in that**, prior to use, it is mixed with the oxidizing agent in a weight ratio of from 5:1 to 1:3.

9. Agent according to Claim 8, **characterized in that** the ready-to-use oxidation colorant has a pH of from 3 to 11.

## Revendications

1. Composition pour la teinture par oxydation de fibres de kératine, **caractérisée en ce qu'**elle contient (a) au moins un dérivé de 4,5-diaminopyrazole de formule (I), (II) ou (III) ou un sel de celui-ci avec des acides organiques ou minéraux 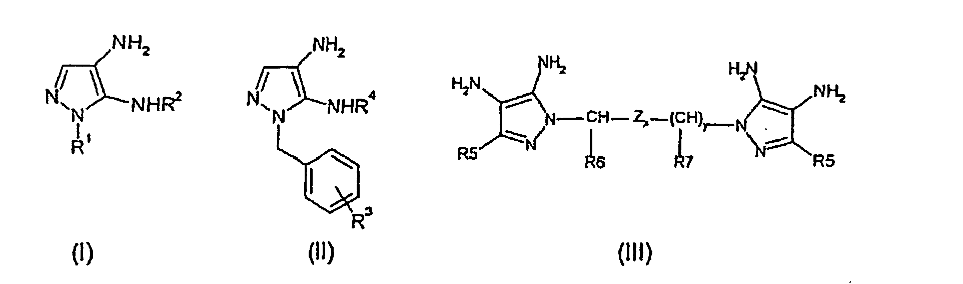
formules dans lesquelles **R1** et **R2** représentent, indépendamment l'un de l'autre, un atome d'hydrogène, un radical alkyle en C₁-C₆ à chaîne droite ou ramifiée, un radical phényle (éventuellement substitué) ou un radical hydroxyalkyle en C₂-C₄ à chaîne droite ou ramifiée ;
**R3** représente un atome d'halogène, un groupe alkyle en C₁-C₄ à chaîne droite ou ramifiée ou un groupe alcoxy en C₁-C₄ à chaîne droite ou ramifiée
et **R4** représente un atome d'hydrogène, un groupe alkyle en C₁-C₄ à chaîne droite ou ramifiée ou un groupe hydroxyalkyle en C₂-C₄ à chaîne droite ou ramifiée ;
**R5** représente un atome d'hydrogène, un groupe alkyle en C₁-C₆ à chaîne droite ou ramifiée, un groupe hydroxyalkyle en C₁-C₄, un groupe aminoalkyle en C₁-C₄, un groupe alkyl(C₁-C₈)amino, un groupe dialkyl(C₁-C₈)amino, un groupe alkyl(C₁-C₄)amino-alkyle(C₁-C₄) ou un groupe dialkyl (C₁-C₄) amino-alkyle (C₁-C₄), un groupe aryle ou un groupe hétéroaryle ;
**R6** et **R7** peuvent être identiques ou différents et représentent un atome d'hydrogène, un groupe alkyle en C₁-C₆ à chaîne droite ou ramifiée, un groupe aryle, un groupe hétéroaryle, un groupe carboxy, un groupe ester d'acide carboxylique, un groupe carboxamido substitué ou non substitué, un groupe hydroxy ou un groupe hydroxyalkyle en C₁-C₄, ou bien R2 et R3 forment ensemble un groupe alkylène en C₁-C₆ (éventuellement substitué) ;
**Z** représente un diradical alkyle en C₁-C₁₀ éventuellement interrompu par un hétéroatome, un diradical aromatique ou hétéroaromatique éventuellement une ou deux fois condensé avec un noyau benzénique et/ou substitué par un groupe hydroxy ou par un groupe alkyle en C₁-C₆, ou un diradical de formule **-Ar-(Alk)**_{**n**}**-Ar-,** dans laquelle **Ar** représente un radical hétéroarylène ou un radical arylène (éventuellement substitué), **Alk** représente un groupe -CH₂- et n représente un nombre entier allant de 0 à 6 ; et
**x** et **y** sont chacun, indépendamment l'un de l'autre, 0 ou 1 ;
ainsi que (b) au moins un dérivé de pyrazol-5-one de formule générale (IV), (V) ou (VI), en particulier (IV) ou (VI) ; ou un sel de celui-ci avec des acides organiques ou minéraux, 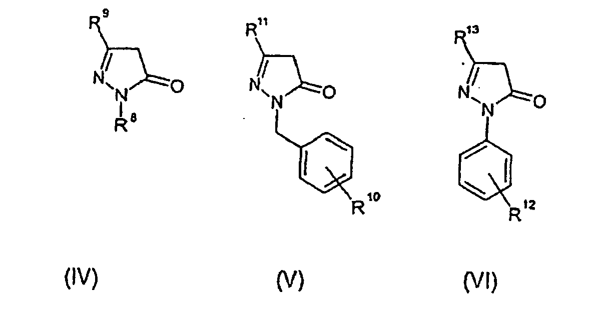 formules dans lesquelles
**R8** représente un groupe alkyle en C₁-C₄ à chaîne droite ou ramifiée, un groupe alcoxy en C₁-C₄ à chaîne droite ou ramifiée, un groupe alcoxy(C₁-C₄)-alkyle(C₁-C₄) à chaîne droite ou ramifiée ou un groupe hydroxyalkyle en C₂-C₄ à chaîne droite ou ramifiée ; et les radicaux **R9** à **R13** représentent chacun, indépendamment les uns des autres, un atome d'hydrogène, un groupe alkyle en C₁-C₄ à chaîne droite ou ramifiée, un groupe hydroxyalkyle en C₂-C₄ à chaîne droite ou ramifiée, un groupe hydroxy, un groupe amino, un groupe alcoxy en C₁-C₄ à chaîne droite ou ramifiée, un groupe alcoxy(C₁-C₄)-alkyle(C₁-C₄) à chaîne droite ou ramifiée ou un groupe hydroxyalkyl(C₂-C₄)amino.

2. Composition selon la revendication 1, **caractérisée en ce que** le dérivé de 4,5-diaminopyrazole de formule (I), (II) ou (III) est choisi parmi le 4,5-diamino-1-méthyl-1H-pyrazole, le 4,5-diamino-1-(4'-méthylbenzyl)pyrazole, le 4,5-diamino-1-(3'-méthylbenzyl)pyrazole, le 4,5-diamino-1-(2'-méthylbenzyl)pyrazole, le 4,5-diamino-1-(2'-hydroxyéthyl)-1H-pyrazole, le 4,5-diamino-1-benzyl-1H-pyrazole, le 4,5-diamino-1-éthyl-1H-pyrazole, le 4,5-diamino-1-isopropyl-1H-pyrazole, le 4,5-diamino-1-pentyl-1H-pyrazole, le 4,5-diamino-1-(4'-méthoxybenzyl)-1H-pyrazole, le 4,5-diamino-1H-pyrazole, le 4,5-diamino-1-(3'-méthoxybenzyl)-1H-pyrazole, le 4,5-diamino-1-(2'-méthoxybenzyl)-1H-pyrazole, le 4,5-diamino-1-(4'-chlorobenzyl)-1H-pyrazole, le 4,5-diamino-1-(3'-chlorobenzyl)-1H-pyrazole, le 4,5-diamino-1-(2'-chlorobenzyl)-1H-pyrazole, le 4-amino-5-méthylamino-1-(4'-méthoxybenzyl)-1H-pyrazole, le 4-amino-5-(2'-hydroxyéthyl)amino-1-(4'-méthoxy-benzyl)-1H-pyrazole, le 4-amino-5-méthylamino-1-(2'-hydroxyéthyl)-1H-pyrazole, le bis(4,5-diaminopyrazol-1-yl)méthane, le 1,2-bis(4,5-diaminopyrazol-1-yl)éthane, le 1,3-bis(4,5-diaminopyrazol-1-yl)propane, le 1,3-bis(4,5-diamino-3-phénylpyrazol-1-yl)propane, le 2,3-bis(4,5-diaminopyrazol-1-yl)propan-1-ol, le N-benzyl-2,3-bis(4,5-diaminopyrazol-1-yl)-propionamide, le 1,3-bis(4,5-diaminopyrazol-1-yl)-cyclohexane, le 1,4-bis(4,5-diaminopyrazol-1-ylméthyl) benzène, le 1,4-bis(4,5-diaminopyrazol-1-ylméthyl)-2,5-diméthoxybenzène, le 1,3-bis(4,5-diaminopyrazol-1-ylméthyl)benzène, le 2,6-bis(4,5-diaminopyrazol-1-ylméthyl)-4-méthylphénol, le 1,2-bis(4,5-diaminopyrazol-1-ylméthyl)benzène, le 1,2-bis(4,5-diaminopyrazol-1-ylméthyl)-4,5-diméthoxy-benzène, le 2,3-bis(4,5-diaminopyrazol-1-yl-méthyl) naphtalène, le 2,3-bis(4,5-diaminopyrazol-1-ylméthyl)anthracène, le 9,10-bis(4,5-diamino-pyrazol-1-ylméthyl)anthracène, le 4,4'-bis(4,5-diaminopyrazol-1-ylméthyl)biphényle, le 1,2-bis[4-(4,5-diaminopyrazol-1-ylméthyl)phényl]éthane, le 2,5-bis(4,5-diaminopyrazol-1-ylméthyl)furanne, le 2,5-bis(4,5-diaminopyrazol-1-ylméthyl)thiophène, le 2,8-bis(4,5-diaminopyrazol-1-ylméthyl)-dibenzothiophène, le 4,4'-bis(4,5-diaminopyrazol-1-ylméthyl)-[2,2']bipyridyle et le 1,2-bis[6-(4,5-diaminopyrazol-1-ylméthyl)pyridin-2-yl]éthane ou leurs sels avec des acides organiques ou minéraux.

3. Composition selon la revendication 1, **caractérisée en ce que** le dérivé de pyrazolone de formule (IV), (V) ou (VI) est choisi parmi la 3-amino-1-phényl-pyrazol-5-one, la 3-méthyl-1-phényl-pyrazol-5-one, la 1,3-diméthyl-pyrazol-5-one, la 3-méthyl-1-(4-sulfényl)-pyrazol-5-one, la 3-méthyl-1-(2-hydroxyéthyl)-pyrazol-5-one et la 1-phényl-pyrazol-5-one ou leurs sels avec des acides organiques ou minéraux.

4. Composition selon l'une quelconque des revendications 1 à 3, **caractérisée en ce qu'**elle contient le 4,5-diaminopyrazole de formule (I), (II) ou (III) et le dérivé de pyrazolone de formule (IV), (V) ou (VI) chacun en une quantité totale de 0,005 à 20 % en poids.

5. Composition selon l'une quelconque des revendications 1 à 4, **caractérisée en ce qu'**en plus des colorants de formule (I) à (VI), elle contient d'autres précurseurs de colorants d'oxydation (développeurs et/ou coupleurs) et/ou des colorants directs.

6. Composition selon la revendication 5, **caractérisée en ce qu'**elle contient les développeurs et coupleurs supplémentaires chacun en une quantité de 0,01 à 20 % en poids.

7. Composition selon la revendication 5, **caractérisée en ce qu'**elle contient les colorants directs en une quantité de 0,1 à 10 % en poids.

8. Composition selon l'une quelconque des revendications 1 à 7, **caractérisée en ce qu'**avant l'emploi on la mélange avec l'oxydant en un rapport pondéral de 5:1 à 1:3.

9. Composition selon la revendication 8, **caractérisée en ce que** la composition de teinture par oxydation prête à l'emploi présente un pH de 3 à 11.
